# EUROPEAN PATENT APPLICATION

(11) **EP 2 508 154 A1**
(43) Date of publication of application: **10.10.2012**
(21) Application number: 09851679.2
(22) Date of filing: 30.11.2009
(51) Int. Cl.: A61F 5/11

(54) **NAIL CORRECTING TOOL**

(71) Applicant: Reflepro Japan Co., Ltd., Osaka-shi, Osaka 542-0081 (JP)
(72) Inventor: YOSHINO, Masafumi, Osaka-shi Osaka 542-0081 (JP); YOSHINO, Narumi, Osaka-shi Osaka 542-0081 (JP)
(74) Representative: Zimmermann, Tankred Klaus
(86) International application number: PCT/JP2009/070124
(87) International publication number: WO 2011/064889

(57) **Abstract**

A corrective brace which has a simple structure with fewer components, prevents lowering of a correcting force of a nail, can be easily attached to the nail body, and will not disturb daily life after attachment is provided.

A nail corrective brace includes:
a first correcting member including:
a first correcting body formed of a hard line material having elasticity and provided with a first locking piece locked by one side end edge in a width direction of a nail body on one end side thereof;
a first correcting operation body formed of a soft line material having elasticity and pulling the first locking piece close to an intermediate portion side in the width direction of the nail body with predetermined tension to pull up the one side end edge of the nail body; and
a first support body which connects the other end side of the first correcting body and one end side of the first correcting operation body and has a support surface brought into contact with a surface of the nail body and supported;

a second correcting member including:
a second correcting body formed of a hard line material having elasticity and provided with a second locking piece locked by the other side end edge in the width direction of the nail body on the one end side thereof;
a second correcting operation body formed of a soft line material having elasticity and pulling the second locking piece close to the intermediate portion side in the width direction of the nail body with predetermined tension to pull up the other side end edge of the nail body; and
a second support body which connects the other end side of the second correcting body and one end side of the second correcting operation body and has a support surface brought into contact with the surface of the nail body and supported; and
a fixing portion which adjusts the tension of pulling up the one end side and the other end side in the width direction of the nail body by crossing the other end side of the first correcting operation body and the other end side of the second correcting operation body with each other at the intermediate portion in the width direction of the nail body and twisting and connects the first correcting operation body and the second correcting operation body in the vicinity of the surface of the nail body while the predetermined tension is maintained.

## Description

### Technical Field

The present invention relates to a nail corrective brace and particularly relates to a nail corrective brace for correcting deformation of a nail such as a so-called ingrown nail in which one of and/or the other side edge ends of a nail bites into a flesh part between the nail and a toe tip so as to roll inward as a toenail grows, for example.

### Background Art

Prior-art methods for correcting ingrown nails as a background of the present invention include a method by a surgical operation for severe deformation of a nail, though it depends on the degree of severity of ingrown nails.
However, since the nail biting into the flesh part is partially incised and removed in the method by a surgical operation, it was difficult to perform a surgical operation if there was a risk of bacterial infection. Even if the ingrown nail is temporarily corrected by partially removing the nail by a surgical operation, the bending characteristic of the entire nail in the rolling direction is not corrected. Therefore, it is highly likely that the ingrown nail phenomenon would occur again after the operation.

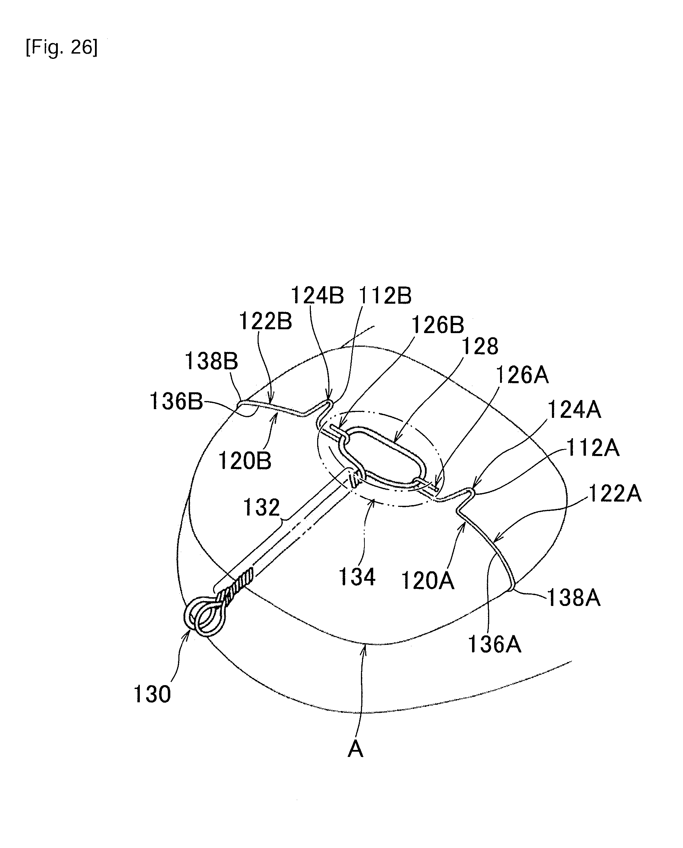

Moreover, in the prior art technology as a background of the present invention, a corrective brace for correcting deformation of a nail which is useful in correcting the deformation of a nail such as an ingrown nail and assisting natural growth of the nail with a simple method without performing a surgical operation is proposed (See Patent Document 1, for example).
As illustrated in Fig. 26, this prior-art corrective brace 100 includes a first correcting body 120A and a second correcting body 120B attached to a nail body A. The first correcting body 120A has a first locking portion 122A locked by one side end edge in the width direction of the nail body A, a first contact portion 124A brought into contact with the surface of the nail body A, and a first connecting hook portion 126A connected to the first contact portion 124A. The second correcting body 120B has a second locking portion 122B locked by the other side end edge in the width direction of the nail body A, a second contact portion 124B brought into contact with the surface of the nail body A, and a second connecting hook portion 126B connected to the second contact portion 124B. A correcting operation portion 128 which pulls up the both side end edges of the nail body A in a direction opposite to the deformation direction is disposed between the first connecting hook portion 126A and the second connecting hook portion 126B.

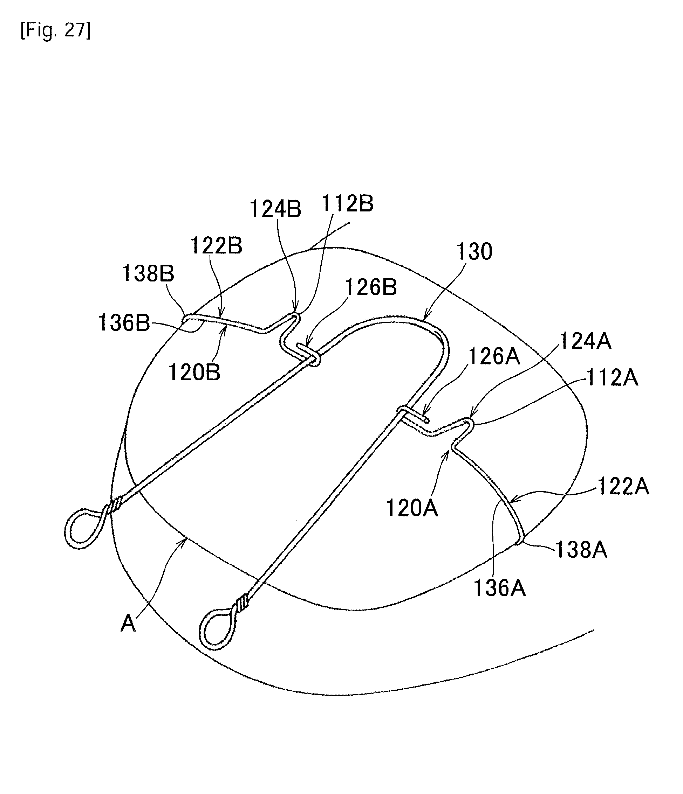

The correcting operation portion 128 is formed of a line material having elasticity and is composed of a wound-up portion 132 formed by crossing one end side and the other end side of a U-shaped tension adjustment member 130 hooked by the first connecting hook portion 126A and the second connecting hook portion 126B with each other as illustrated in Fig. 27 and by winding them up as illustrated in Fig. 26. In this case, a ring-shaped correcting operation portion 128 is formed between the first connecting hook portion 12 6A and the second connecting hook portion 126B while tensions of pulling up the one end side and the other end side in the width direction of the nail body A by the wound-up portion 132 is adjusted.
Furthermore, in this prior-art corrective brace 100, as illustrated in Fig. 26, for example, a covering portion 134 covers the ring-shaped correcting operation portion 128 at the center and the first connecting hook portion 126A, the second connecting hook portion 126B, and the wound-up portion 132 in the periphery of the correcting operation portion 128. The covering portion 134 is formed by applying an ultraviolet curable resin material on the above-described predetermined portions and then, by irradiating the ultraviolet rays to the applicable portions for a predetermined time.

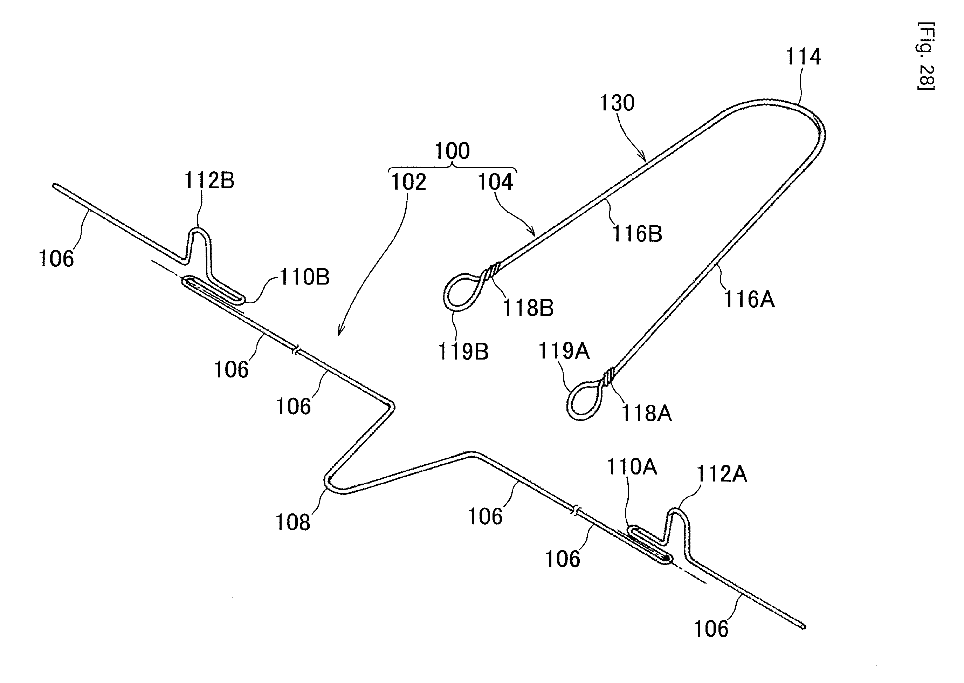

As illustrated in Fig. 28, for example, this prior-art corrective brace 100 is composed of two components, that is, a base material 102 for the correcting body and a base material 104 for the correcting operation portion.
The base material 102 for the correcting body includes a base line material 106 having a length direction. A substantially U-shaped projecting portion 108 is formed at a middle part in the length direction of the base line material 106. The projecting portion 108 projects in a direction orthogonal to the axial direction of the base line material 106. An S-shaped bent portion 110A is formed between one end in the length direction of the base line material 106 and the projecting portion 108. The S-shaped bent portion 110A is arranged on the same plane as the projecting portion 108 and its one end extends in a direction opposite to the projecting direction of the projecting portion 108. Similarly, an S-shaped bent portion 110B is formed between the other end in the length direction of the base line material 106 and the projecting portion 108.

Moreover, an inverted U-shaped bent portion 112A, for example, is formed between one end in the length direction of the base line material 106 and the one bent portion 110A. The inverted U-shaped bent portion 112A is connected to one end of the above-described S-shaped bent portion 110A. The inverted U-shaped bent portion 112A is arranged on the plane orthogonal to the plane on which the projecting portion 108 and the S-shaped bent portion 110A are arranged. In this case, the inverted U-shaped bent portion 112A extends in a direction perpendicular to the axial direction of the base line material 106. Similarly, an inverted U-shaped bent portion 112B is formed at one end of the S-shaped bent portion 110B between the other end in the length direction of the base line material 106 and the other S-shaped bent portion 110B.
The base line material 106, the projecting portion 108, the S-shaped bent portions 110A and 110B, and the inverted U-shaped bent portions 112A and 112B are integrally formed of a metal material having elasticity such as spring steel or the like.

Regarding the above-described base material 102 for the correcting body, the left and right S-shaped bent portions 110A and 110B and the inverted U-shaped bent portions 112A and 112B of the initial base line material 106 are positioned by directing the projecting portion 108 to the toe tip and then, by erecting it upward substantially perpendicularly. That is, when the projecting portion 108 is directed to the toe tip, the inverted U-shaped bent portions 112A and 112B are directed to the root (nail root) side of the nail body A in the opposite direction. This projecting portion 108 has a function of instructing arrangement direction so as to clarify the arrangements and directions of the S-shaped bent portions 110A and 110B and the inverted U-shaped bent portions 112A and 112B forming the first correcting body 120A and the second correcting body 120B.

On the other hand, as illustrated in Fig. 28, the base material 104 for the correcting operation portion includes the tension adjustment member 130 made of a line material and having a U-shape on a plan view. The tension adjustment member 130 has an arc-shaped curved portion 114, for example, at the center portion in the length direction. Linear-shaped leg portions 116A and 116B are formed between one end part of the tension adjustment member 130 and the curved portion 114 and between the other end part of the tension adjustment member 130 and the curved portion 114, respectively. Furthermore, ring-shaped insertion rings 119A and 119B are formed at the one end part and the other end part of the tension adjustment member 130 through twisted portions 118A and 118B, respectively. The tension adjustment member 130, the twisted portions 118A and 118B, and the insertion rings 119A and 119B are integrally formed of a metal material having elasticity such as a spring steel or the like, similarly to the above-described base material 12 for the correcting body.

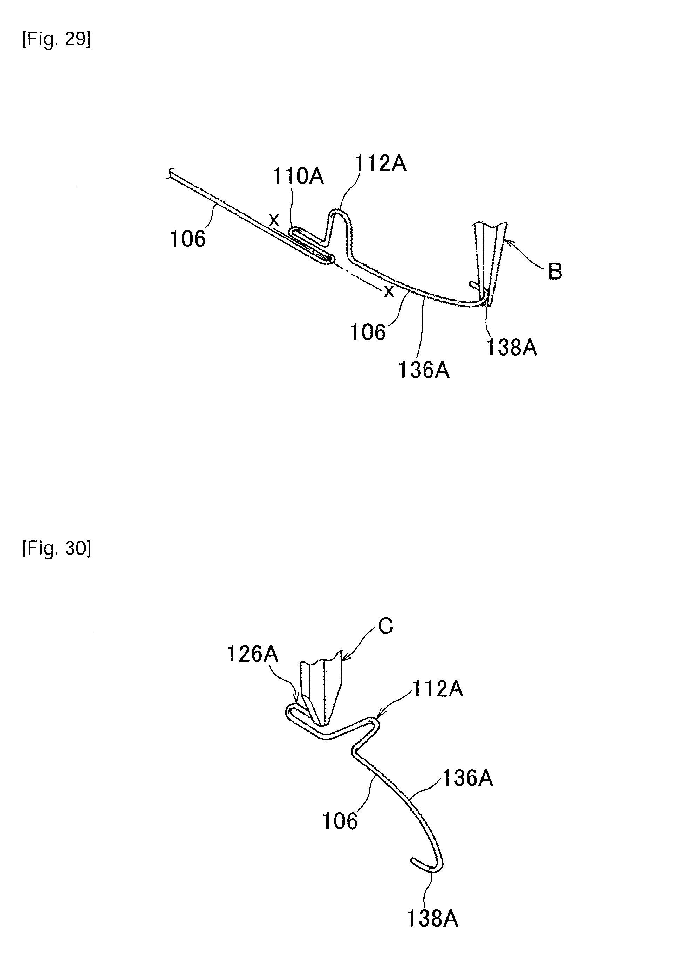

Subsequently, a method of forming the first correcting body 120A and the second correcting body 120B from the base material 102 for the correcting body will be described.
First, the base material 102 for the correcting body is held by a hand with the projecting portion 108 directed upward, and the S-shaped bent portion 110A is sandwiched by a needle holder (not shown) and fixed.
Subsequently, one side in the length direction of the base line material 106 is conformed to the size and curve of the nail body A by pliers and the like so as to be gently curved to form a curve 136A as illustrated in Figs. 26 and 27. Then, an end part of the curve 136A is cut by a cutter (not shown) in accordance with the width of the nail body A and then, the curved degree of the curve 136A is adjusted in accordance with the rolling degree of the nail body A. Moreover, by sandwiching a spot approximately 5 mm, for example, from the distal end of the curve 136A by means of bending means B such as pliers or the like and bending it inward as illustrated in Fig. 29, a U-shaped bent portion 138A is formed.
Then, by cutting a spot approximately 1 mm, for example, from the distal end of the bent portion 138A by means of a cutter (not shown), the first locking portion 122A having the curve 136A and the bent portion 138A on one side in the length direction of the base line material 106 is formed.
Then, the S-shaped bent portion 110A is cut at a one-dot chain line x-x portion illustrated in Fig. 29 and then, a spot approximately 0.5 mm from the cut end part is cut by cutting means C such as a cutter or the like as illustrated in Fig. 30 so as to form the first connecting hook portion 126A. At the same time, the inverted U-shaped bent portion 112A becomes the first contact portion 124A. As described above, the first correcting body 120A composed of the first locking portion 122A, the first contact portion 124A, and the first connecting hook portion 126A is formed.
Similarly, on the other side in the length direction of the base line material 106, as illustrated in Fig. 26, the second locking portion 122B having a curve 136B and a bent portion 138B is formed, the second connecting hook portion 126B and the second contact portion 124B are also formed, and the second correcting body 120B composed of the second locking portion 122B, the second contact portion 124B, and the second connecting hook portion 126B is formed.

### Citation List

### Patent Literature

Patent Document 1: Japanese Patent No. 3530901

### Summary of Invention

### Technical Problem

However, with the prior-art corrective brace 100 as illustrated in Patent Document 1, for example, the force of pulling up the one end side and the other end side in the width direction of the nail body A or in this case, the tension acting on the first connecting hook portion 126A and the second connecting hook portion 126B acts through the ring-shaped correcting operation portion 128 as illustrated in Figs. 26 and 27. That is, the tension acts indirectly on the first connecting hook portion 126A and the second connecting hook portion 126B via the correcting operation portion 128. Thus, it is concerned that the tension might lower. If the tension lowers, the corrective brace 100 becomes loose and comes off from the nail body A.

Moreover, since this prior-art corrective brace 100 has a structure that the first connecting hook portion 126A and the second connecting hook portion 126B disposed in the upright state with respect to the surface of the nail body A are hooked by the tension adjustment member 130, the correcting operation portion 128 floats above the surface of the nail body A by line diameters of the first connecting hook portion 126A and the second connecting hook portion 126B. In this case, the position of the correcting operation portion 128 becomes high with respect to the surface of the nail body A and in a state in which a so-called a projection is formed. As a result, when a sock or a shoe is worn, a sense of discomfort or a rip in the sock by the end of the projection and the like may be caused by the sock coming into contact with the projection, thereby disrupting daily life.

Furthermore, with this prior-art corrective brace 100, as illustrated in Fig. 26, for example, the first connecting hook portion 126A, the second connecting hook portion 126B, and the wound-up portion 132 in the periphery of the correcting operation portion 128 around the ring-shaped correcting operation portion 128 are covered by the covering portion 134. Thus, the covering region becomes large, an amount of a covering material (an ultraviolet curable resin material, for example) used for the covering portion 134 becomes large, and a material cost becomes high. In this case, the use amount of the covering material used in the covering portion 134 becomes larger by the increase in the height of the correcting operation portion 128 from the surface of the nail body A, and so the material cost of the covering portion 134 further rises.

Moreover, with this prior-art corrective brace 100, two components, that is, the base material 102 for the correcting body and the base material 104 for the correcting operation portion are needed in order to form the first correcting body 120A, the second correcting body 120B, and the correcting operation portion 128 as illustrated in Fig. 28. Moreover, machining processes including a process of curving, cutting, bending, folding and the like of the base line material 106 are applied to the base material 102 for the correcting body as described in the above "Background Art" in order to form particularly the first and second locking portions 122A and 122B, the first and second contact portions 124A and 124B, and the first and second connecting hook portions 126A and 126B. Furthermore, the machining processes are complicated including many steps and also require high-level technologies. Therefore, the method of mounting this prior-art corrective brace 100 to the nail body A also requires complicated and high-level skills and takes a long time.

Moreover, with this prior-art corrective brace 100, as illustrated in Fig. 28, the inverted U-shaped bent portion 112A which becomes the first contact portion 124A, the inverted U-shaped bent portion 112B which becomes the second contact portion 124B, the S-shaped bent portion 110A which becomes the first connecting hook portion 126A, and the S-shaped bent portion 110B which becomes the second connecting hook portion 126B each have their arrangements and directions determined in advance in the base material 102 for the correcting body.
Therefore, with this prior-art corrective brace 100, if the first correcting body 120A fits the nail body A but the second correcting body 120B does not fit the nail body A, it is necessary to fabricate the second correcting body 120B again by using another base material 102 for the correcting body. That is, even if only either one of the first correcting body 120A and the second correcting body 120B does not fit the nail body A, another base material 102 for the correcting body is required. Thus, with this prior-art corrective brace 100, a manufacturing cost is high and hence, time for mounting the corrective brace 100 takes even longer.

Therefore, a major object of the present invention is to provide a corrective brace which has a simple structure with fewer components, prevents a correcting force acting on a nail from lowering, can be easily attached to the nail body, and will not disturb daily life after attachment.

### Solution to Problem

The present invention according to claim 1 is a nail corrective brace, including:
a first correcting member including:
   a first correcting body formed of a hard line material having elasticity and provided with a first locking piece locked by one side end edge in a width direction of a nail body on one end side thereof;
   a first correcting operation body formed of a soft line material having elasticity and pulling the first correcting body close to an intermediate portion side in the width direction of the nail body with predetermined tension to pull up the one side end edge of the nail body by the first locking piece; and
   a first support body which connects the other end side of the first correcting body and one end side of the first correcting operation body and has a pressing surface brought into contact with the surface of the nail body to press the nail body downward;
a second correcting member including:
   a second correcting body formed of a hard line material having elasticity and provided with a second locking piece locked by the other side end edge in the width direction of the nail body on the one end side thereof;
   a second correcting operation body formed of a soft line material having elasticity and pulling the second correcting body close to the intermediate portion side in the width direction of the nail body with predetermined tension to pull up the other side end edge of the nail body by the second locking piece; and
   a second support body which connects the other end side of the second correcting body and one end side of the second correcting operation body and has a pressing surface brought into contact with the surface of the nail body to press the nail body downward; and
   a fixing portion which adjusts the tension of pulling up the one end side and the other end side in the width direction of the nail body by crossing the other end side of the first correcting operation body and the other end side of the second correcting operation body with each other at the intermediate portion in the width direction of the nail body and twisting, and connects the first correcting operation body and the second correcting operation body in the vicinity of the surface of the nail body while the predetermined tension is maintained.

In the present invention according to claim 1, in the first correcting member and the second correcting member, the first locking piece of the first correcting body and the second locking piece of the second correcting body are locked by one end edge portion and the other end edge portion in the width direction of a nail body of an ingrown nail or the like, respectively. The first correcting body is connected to the first correcting operation body through the first support body, while the second correcting body is connected to the second correcting operation body through the second support body. The first support body and the second support body are brought into contact with the surface of the nail body and supported, respectively.

The first correcting operation body and the second correcting operation body are crossed with each other at the intermediate portion in the width direction of the nail body and twisted. At this time, the first correcting operation body and the second correcting operation body pull the first correcting body and the second correcting body to the intermediate portion side in the width direction of the nail body with predetermined tension and pull up the one end edge portion and the other end edge portion of the nail body by the first locking piece and the second locking piece, respectively. That is, the one end edge portion and the other end edge portion in the width direction of the nail body are pulled by the first correcting operation body and the second correcting operation body in a direction (upward direction) opposite to the rolling direction of the ingrown nail and pulled up.

The longer a twisting length of the first correcting operation body and the second correcting operation body is, that is, the more they are twisted (the number of twisting turns), the larger this tension can be, while on the contrary, the shorter the twisting length is, that is, the less they are twisted (the number of twisting turns), the smaller this tension can be. Therefore, this tension is adjusted as appropriate in accordance with the size and shape of the nail body, the curved degree of the ingrown nail and the like.

Moreover, the pressing surfaces of the first support body and the second support body are brought into contact with the surface of the nail body and press the nail body downward. Thus, the first correcting body, the first correcting operation body, the second correcting body, and the second correcting operation body are stably supported by the first support body and the second support body on the surface of the nail body, respectively, without shifting.

The first correcting operation body and the second correcting operation body are twisted while the tension is adjusted and connected/fixed by the fixing portion to the vicinity of the surface of the nail body while the predetermined tension is maintained. Thus, the tension in the direction (upward direction) opposite to the rolling direction of the ingrown nail continuously acts on the both end edges in the width direction of the ingrown nail and prevents lowering of the tension. Therefore, according to the nail corrective brace of the present invention according to claim 1, deformation of the nail such as ingrown nails can be corrected.

In the present invention according to claim 1, since the first correcting operation body and the second correcting operation body are constructed to be connected/fixed by being directly crossed with each other at the intermediate portion in the width direction of the nail body and twisted, the tension can be increased as compared with Patent Document 1 (prior art), for example, in which the above-described tension acts via the ring-shaped correcting operation portion. Thus, a connecting force between the first correcting operation body and the second correcting operation body can be stably and continuously maintained.

Moreover, the present invention according to claim 1 is not constructed such that the tension adjustment member is hooked by the first connecting hook portion and the second connecting hook portion disposed in the upright state with respect to the surface of the nail body as illustrated in Patent Document 1 (prior art), for example. Thus, the height of the fixing portion which becomes a connection end portion between the first correcting operation body and the second correcting operation body from the surface of the nail body can be lowered as compared with Patent Document 1 (prior art), and does not disturb daily life.

Furthermore, with the present invention according to claim 1, a pair of correcting members can be formed of two members, that is, the first correcting member and the second correcting member, and so the number of components is smaller than a tool requiring at least three members, that is, the first correcting body, the second correcting body, and the tension adjustment member forming the ring-shaped correcting operation portion as illustrated in Patent Document 1 (prior art). Moreover, with the present invention according to claim 1, the number of machining processes including the process of curving, cutting, bending, folding and the like of the base line material for forming the first correcting member and the second correcting member is extremely fewer than Patent Document 1 (prior art) and the machining processes are not complicated or require high-level technologies. Thus, the method of mounting the first correcting member and the second correcting member of the present invention according to claim 1 on the nail body is simple, and mounting time can be reduced.

Moreover, in the corrective brace illustrated in Patent Document 1 (prior art), as illustrated in Fig. 28, arrangements and directions of the members constituting the first correcting body and the second correcting body are determined in advance, respectively, in the base material for the correcting body, and if even only either one of the first correcting body and the second correcting body does not fit the nail body, another base material for the correcting body is required. On the contrary, in the present invention according to claim 1, each of the first correcting member and the second correcting member is formed of a single unit, the directions and arrangements when being mounted on the nail body are not determined in advance, and the first correcting member and the second correcting member can replace each other in use. Thus, manufacturing costs of the first correcting member and the second correcting member is lower and time required for mounting the first correcting member and the second correcting member on the nail body can be further reduced as compared with the corrective brace illustrated in Patent Document 1 (prior art).

The present invention according to claim 2 is an invention dependent on the invention according to claim 1 and is a nail corrective brace in which
the first support body includes a first plate-shaped main body and a first chamfered portion disposed in the first plate-shaped main body;
the other end side of the first correcting body and the one end side of the first correcting operation body are fitted in the first chamfered portion;
the first correcting body and the first correcting operation body are connected through the first support body by welding with the first chamfered portion or welding the other end side of the first correcting body and the one end side of the first correcting operation body with the first chamfered portion together;
the second support body includes a second plate-shaped main body and a second chamfered portion disposed in the second plate-shaped main body;
the other end side of the second correcting body and the one end side of the second correcting operation body are fitted in the second chamfered portion; and
the second correcting body and the second correcting operation body are connected through the second support body by welding with the second chamfered portion or welding the other end side of the second correcting body and the one end side of the second correcting operation body with the second chamfered portion together.

Since the present invention according to claim 2 has the above-described configuration, a connection area between the first chamfered portion disposed in the first plate-shaped main body and the other end side of the first correcting body and the one end side of the first correcting operation body fitted in the first chamfered portion can be ensured wider than the connection by merely welding the other end side of the first correcting body and the one end side of the first correcting operation body, for example, and welding strength can be improved. Thus, the first correcting body and the first correcting operation body are firmly connected to each other through the first support body. Similarly, the second correcting body and the second correcting operation body are firmly connected to each other through the second support body.

The present invention according to claim 3 is an invention dependent on the invention according to claim 2 and is a nail corrective brace described in claim 2, in which:
the first support body further includes a first recess portion communicating with the first chamfered portion;
a first diameter-enlarged flat portion is disposed at the other end of the first correcting body and one end of the first correcting operation body, respectively;
the other end side of the first correcting body and the one end side of the first correcting operation body are fitted in the first chamfered portion so that the both first diameter-enlarged flat portions are fitted in the first recess portion and are welded at least to the first chamfered portion and connected;
the second support body further includes a second recess portion communicating with the second chamfered portion;
a second diameter-enlarged flat portion is disposed at the other end of the second correcting body and one end of the second correcting operation body, respectively;
the other end side of the second correcting body and the one end side of the second correcting operation body are fitted in the second chamfered portion so that the both second diameter-enlarged flat portions are fitted in the second recess portion and are welded at least to the second chamfered portion and connected.

Since the present invention according to claim 3 has the above-described configuration, if the first diameter-enlarged flat portion is fitted in the first chamfered portion, the first diameter-enlarged flat portion can be locked by the first recess portion. Therefore, particularly the other end of the first correcting body and the one end of the first correcting operation body can be fitted, positioned and welded in the first chamfered portion more easily than the invention according to claim 2, for example. Similarly, the other end of the second correcting body and the one end of the second correcting operation body can be easily fitted, positioned and welded in the second chamfered portion.

The present invention according to claim 4 is an invention dependent on the invention according to claim 1 and is a nail corrective brace, in which:
the first support body further includes a first tunnel hole penetrating the first support body from one end to the other end and a first stepped portion crossing the first tunnel hole substantially at a right angle on a plan view;
a first retaining portion is disposed at the other end of the first correcting body and the one end of the first correcting operation body, respectively;
the first correcting body and the first correcting operation body are inserted so that the sides opposite to the first retaining penetrate from the first stepped portion sides of the one first tunnel hole and the other first tunnel hole to the one end and the other end of the first support body, respectively;
the first retaining portion is welded to the first stepped portion while being locked by a peripheral edge of the first tunnel hole on the first stepped portion side;
the second support body further includes a second tunnel hole penetrating the second support body from one end to the other end and a second stepped portion crossing the second tunnel hole substantially at a right angle on a plan view;
a second retaining portion is disposed at the other end of the second correcting body and the one end of the second correcting operation body, respectively;
the second correcting body and the second correcting operation body are inserted so that the sides opposite to the second retaining penetrate from the second stepped portion sides of the one second tunnel hole and the other second tunnel hole to the one end and the other end of the second support body, respectively; and
the second retaining portion is welded to the second stepped portion while being locked by a peripheral edge of the second tunnel hole on the second stepped portion side.

Since the present invention according to claim 4 has the above-described configuration, if the first correcting body is inserted into one of the first tunnel holes and the first correcting operation body is inserted into the other first tunnel hole, one of the first retaining portions is locked by the peripheral edge of the one tunnel hole on the first stepped portion side and the other first retaining is locked by the peripheral edge of the other tunnel hole on the first stepped portion side. Thus, the other end of the first correcting body and the one end of the first correcting operation body can be inserted, positioned and welded into the first support body more easily than the invention according to claim 2 or 3, for example. After the welding, the first correcting body and the first correcting operation body are retained and firmly connected to the first support body. Similarly, the other end of the second correcting body and the one end of the second correcting operation body can be easily inserted, positioned and welded into the second support body. After the welding, the second correcting body and the second correcting operation body are retained and firmly connected to the second support body.

The present invention according to claim 5 is an invention dependent on any one of claims 1 to 4 and is a nail corrective brace further including a covering portion which covers the fixing portion.

In the present invention according to claim 5, the covering portion is formed so as to cover the fixing portion, and thus, the fixing portion which connects the first correcting operation body and the second correcting operation body in the vicinity of the surface of the nail body and a peripheral connection end portion thereof are protected, and the connecting force between the first correcting member and the second correcting member is made firmer, so that the connecting force is stably continued/maintained.

Moreover, in the present invention according to claim 5, the covering portion is formed so as to cover the fixing portion, and thus, the amount of a covering material (an ultraviolet curable resin material, for example) used for the covering portion becomes smaller than the covering portion formed over a wide range of the ring-shaped correcting operation portion, the first connecting hook portion, and the second connecting hook portion as illustrated in Patent Document 1 (prior art), for example, and a material cost can be low. In this case, since the height of the fixing portion which becomes the connection end portion between the first correcting operation body and the second correcting operation body from the surface of the nail body can be lowered, the use amount of the covering material used in the covering portion is further reduced by the height lowered. Accordingly, the material cost of the covering portion can be low.

The present invention according to claim 6 is an invention dependent on the invention according to claim 5 and is a nail corrective brace in which the covering portion includes a decoration portion to which a decoration technology is applied.

In the present invention according to claim 6, various decoration technologies are applied to the covering portion and a decoration portion is formed, and thus, its appearance becomes excellent, and particularly women can have their nails corrected casually with a sense of enjoying fashion.

### Advantageous Effects of Invention

According to the present invention, a nail corrective brace, which has a simple structure with fewer components, prevents lowering of a correcting force acting on a nail, is easy to be attached to the nail body, and will not disturb daily life after attachment can be obtained.

The above-described objects, other objects, features, and advantages of the present invention will be made further obvious from the detailed description of embodiments of the invention below by referring to the attached drawings.

### Brief Description of Drawings

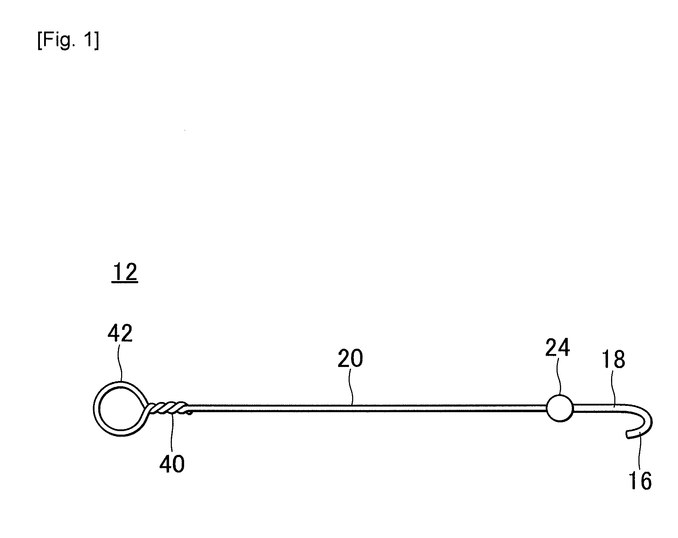

Fig. 1 is a perspective view illustrating an example of a first correcting member of a nail corrective brace according to the present invention.

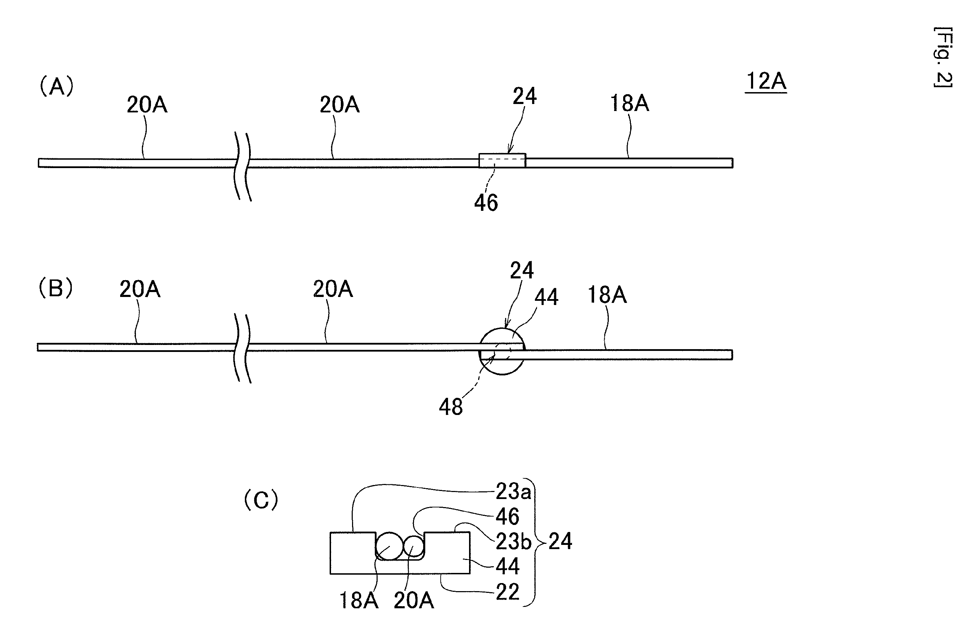

Figs. 2 are views illustrating an example of a base material of the first correcting member illustrated in Fig. 1, in which Fig. 2A is a front view, Fig. 2B is a plan view, and Fig. 2C is a right side view.

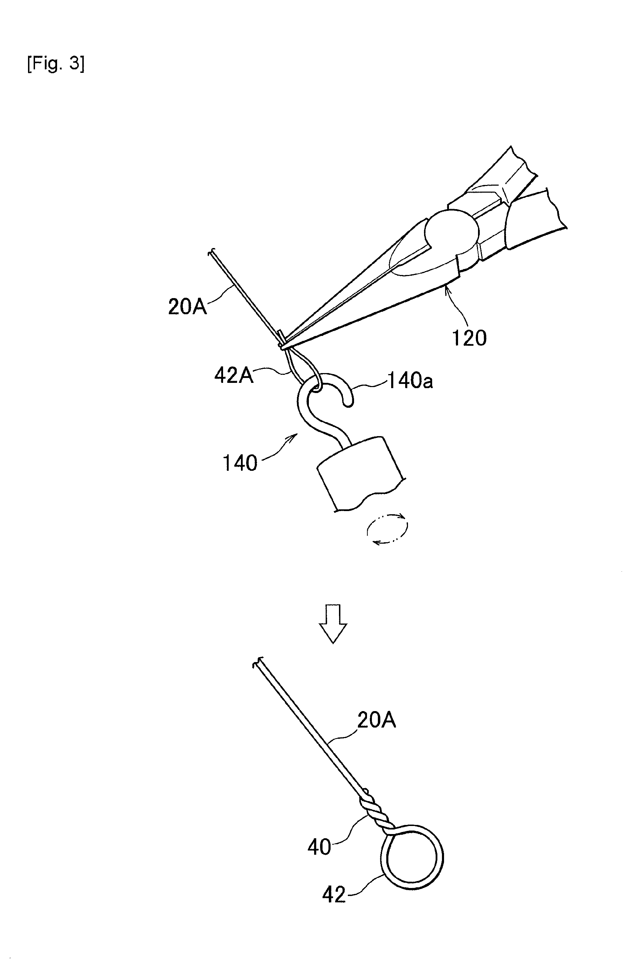

Fig. 3 is a perspective view of an essential part illustrating an example of a machining process for forming a first insertion ring in the base material illustrated in Fig. 2.

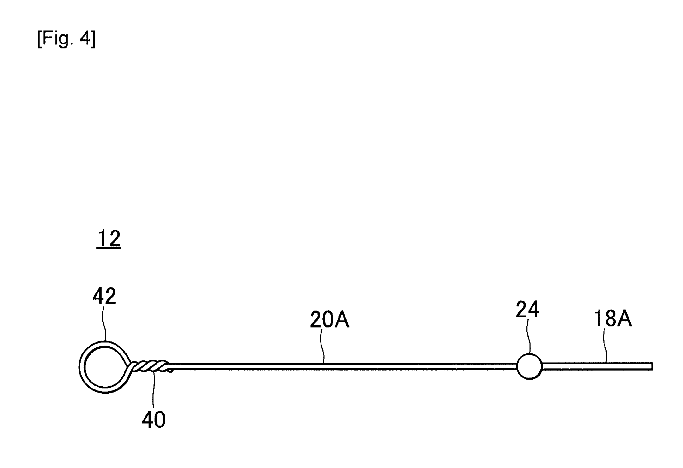

Fig. 4 is a perspective view illustrating a mode in which the first insertion ring is formed in the base material illustrated in Fig. 2 through the machining process illustrated in Fig. 3.

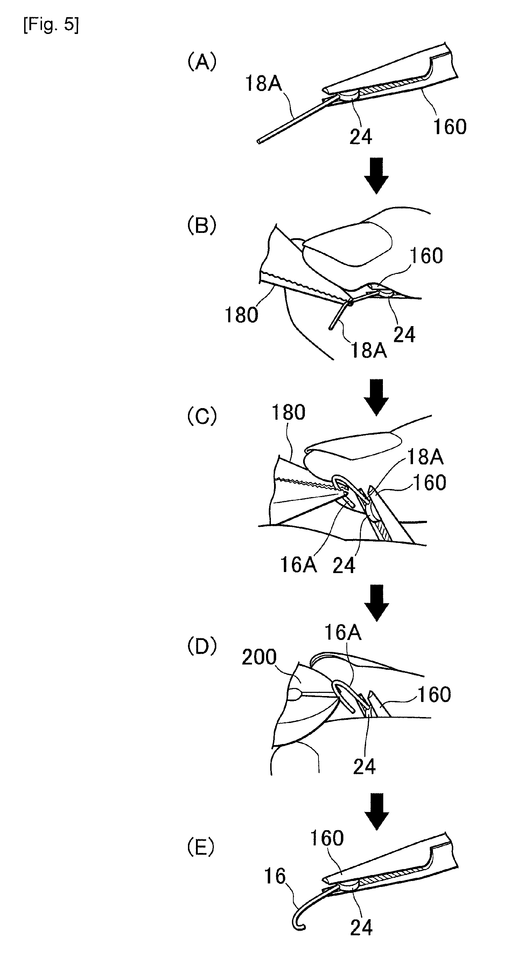

Fig. 5 is a process diagram illustrating, by means of a perspective view of an essential part, an example of the machining process for forming a first locking piece in the base material in which the first insertion ring illustrated in Fig. 4 is formed.

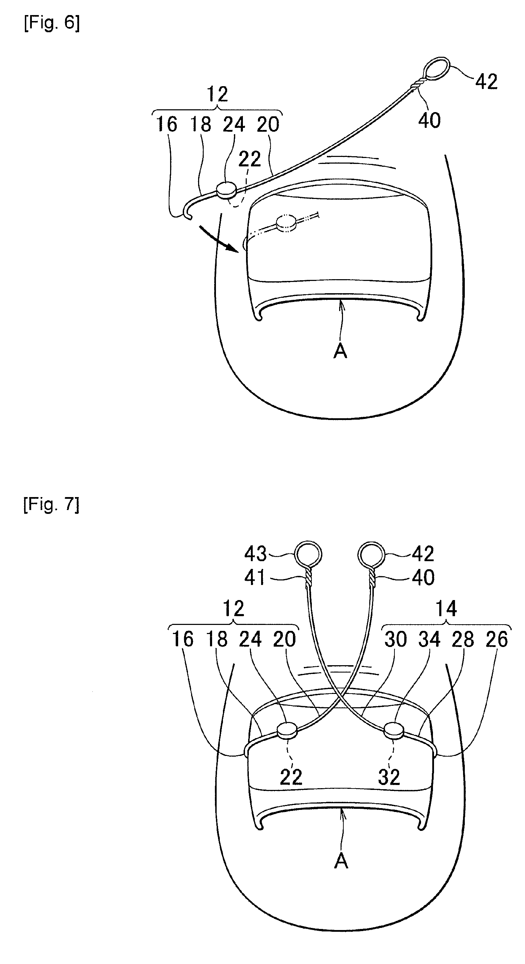

Fig. 6 is a perspective view of an essential part illustrating a process in the middle of mounting of the first correcting member by locking the first locking piece by one side end edge of the nail body.
Fig. 7 is a perspective view of an essential part illustrating a process in the middle of mounting of a second correcting member by locking a second locking piece by the other side end edge of the nail body subsequently to the process illustrated in Fig. 6.

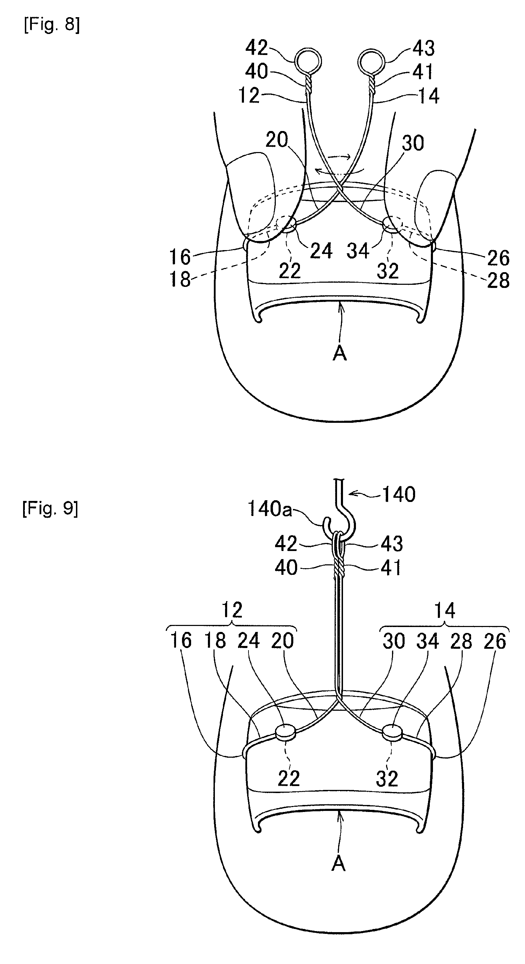

Fig. 8 is a perspective view of an essential part illustrating a process in the middle of twisting the first correcting member and the second correcting member subsequently to the process illustrated in Fig. 7.
Fig. 9 is a perspective view of another essential part illustrating a process in the middle of twisting the first correcting member and the second correcting member.

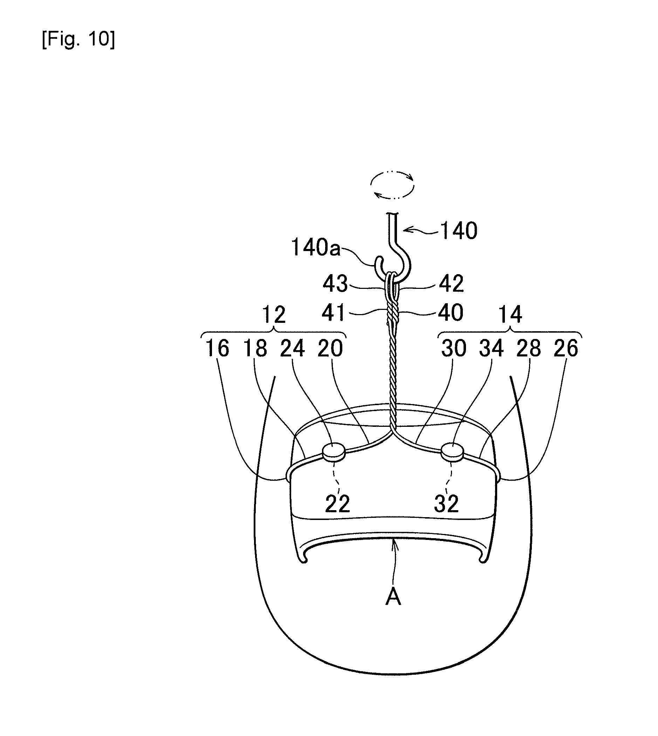

Fig. 10 is a perspective view of another essential part illustrating a process of twisting the first correcting member and the second correcting member.

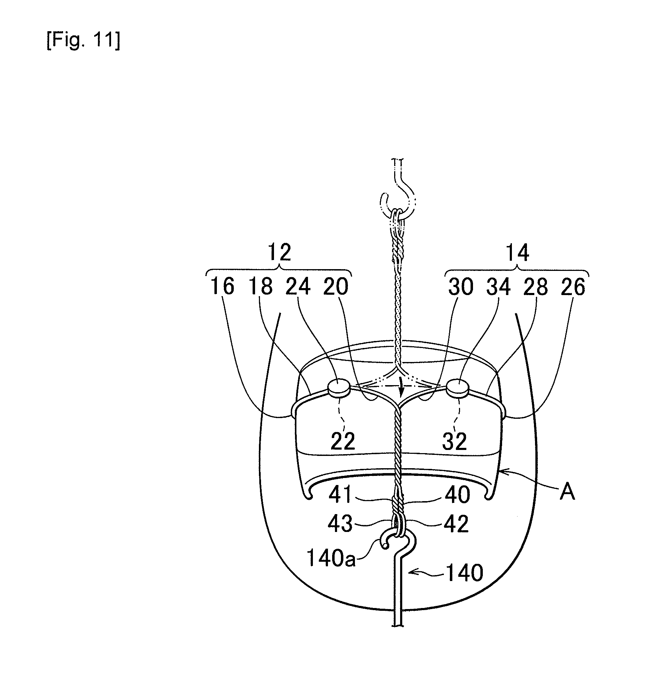

Fig. 11 is a perspective view of still another essential part illustrating a process of twisting the first correcting member and the second correcting member.

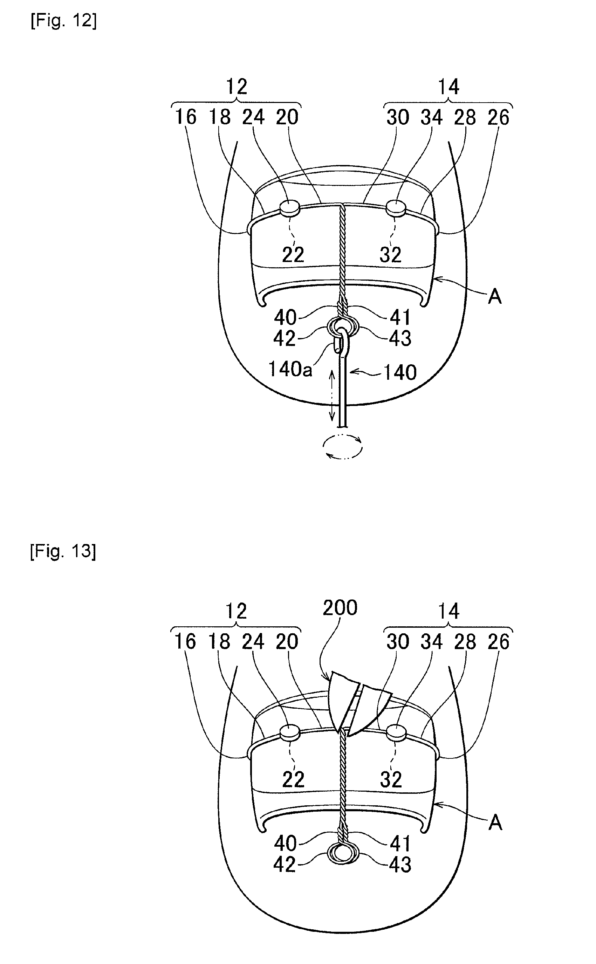

Fig. 12 is a perspective view of still another essential part illustrating a process of twisting the first correcting member and the second correcting member.
Fig. 13 is a perspective view of an essential part illustrating a process of forming a fixing portion after the process of twisting the first correcting member and the second correcting member is finished.

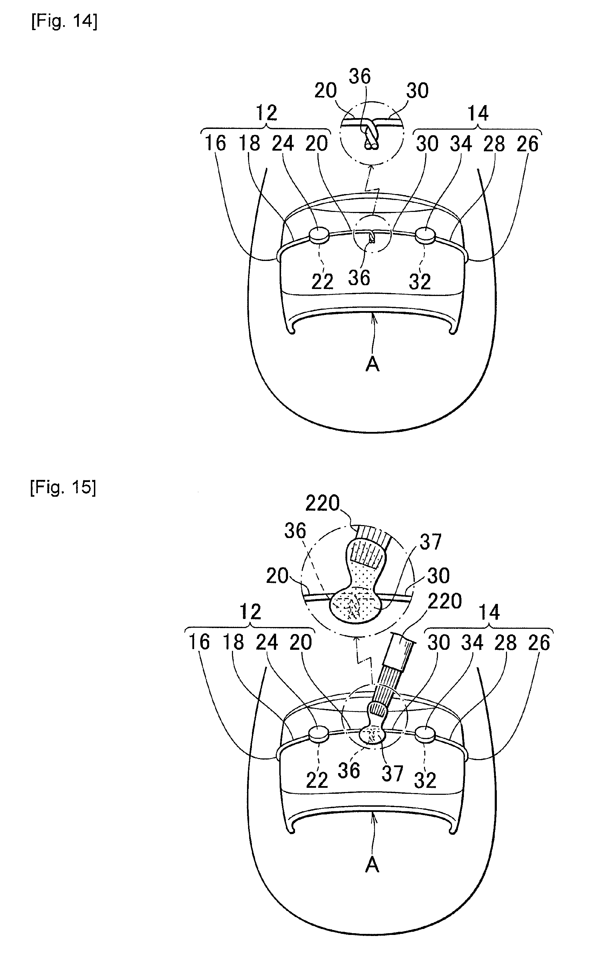

Fig. 14 is a perspective view of an essential part illustrating a mode in which the fixing portion has been formed through the process illustrated in Fig. 13.
Fig. 15 is a perspective view of an essential part illustrating an example of a process of applying a covering material which covers the fixing portion.

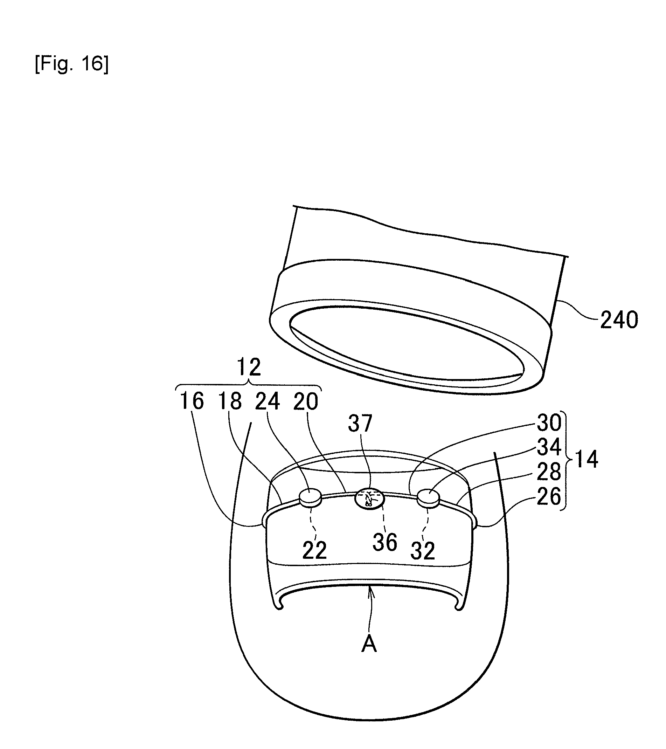

Fig. 16 is a perspective view of an essential part illustrating an example of a process of curing the portion applied with the covering material subsequently to the process illustrated in Fig. 15.

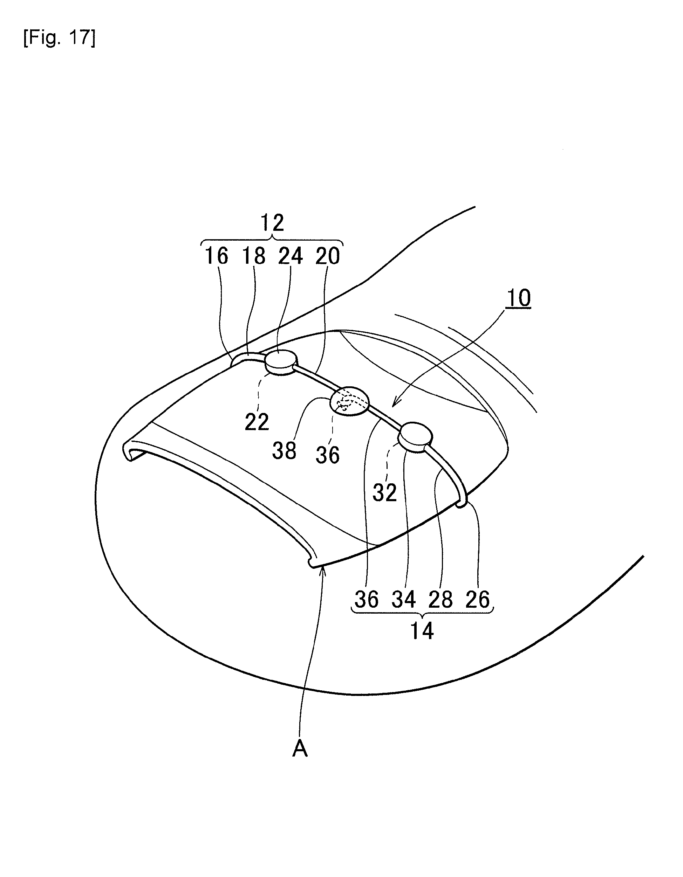

Fig. 17 illustrates a mode in which the covering portion is formed through the process illustrated in Fig. 16 and is a perspective view of an essential part illustrating a use state of the nail corrective brace according to the present invention.

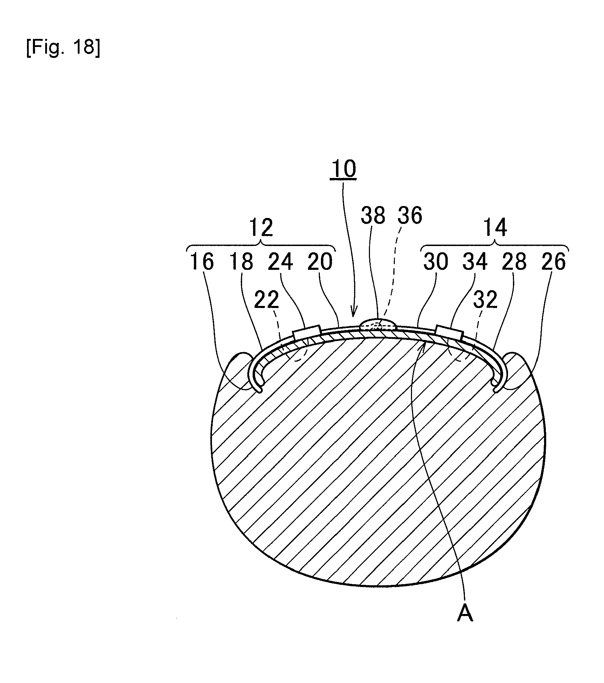

Fig. 18 illustrates a mode in which the first correcting member and the second correcting member are mounted on the nail body and the covering portion has been formed on the fixing portion through the processes illustrated in Figs. 6 to 16 and is a sectional view of an essential part illustrating the use state of the nail corrective brace according to the present invention.

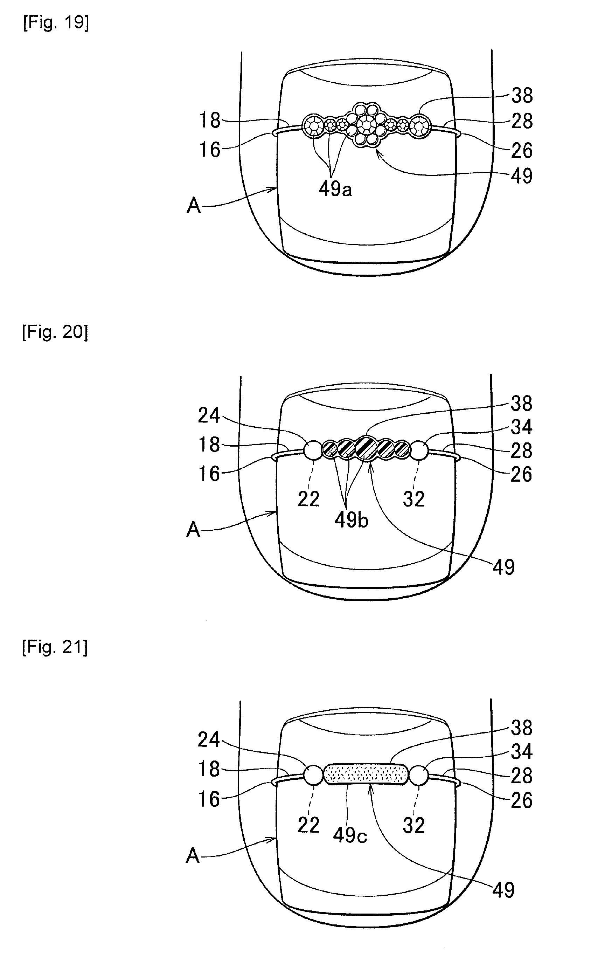

Fig. 19 is a perspective view of an essential part illustrating an example of a mode in which a decoration portion is formed on the covering portion.
Fig. 20 is a perspective view of an essential part illustrating another example of a mode in which a decoration portion is formed on the covering portion.
Fig. 21 is a perspective view of an essential part illustrating still another example of a mode in which a decoration portion is formed on the covering portion.

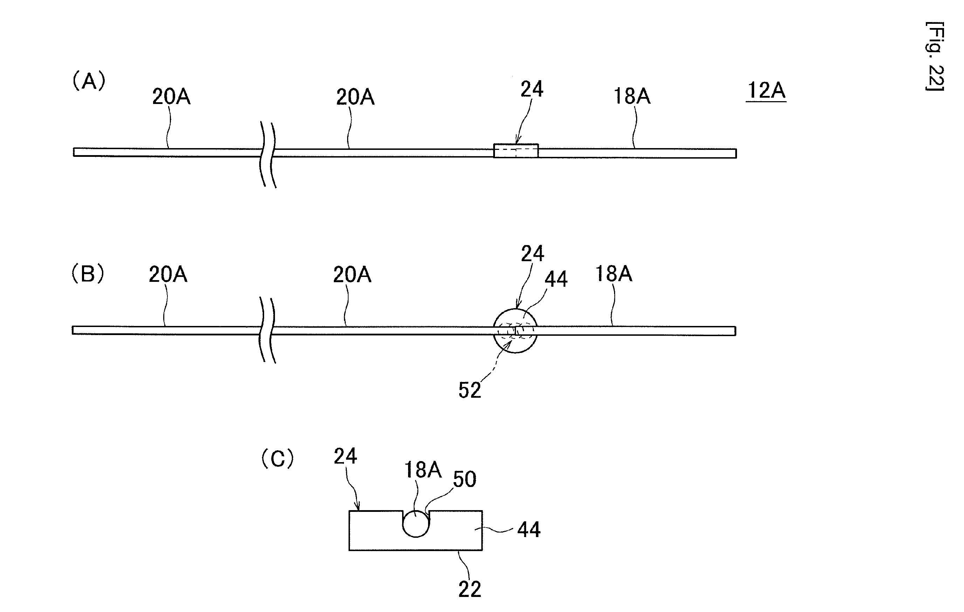

Figs. 22 are views illustrating another example of the base material of the first correcting member illustrated in Fig. 1, in which Fig. 22A is a front view, Fig. 22B is a plan view, and Fig. 22C is a right side view.

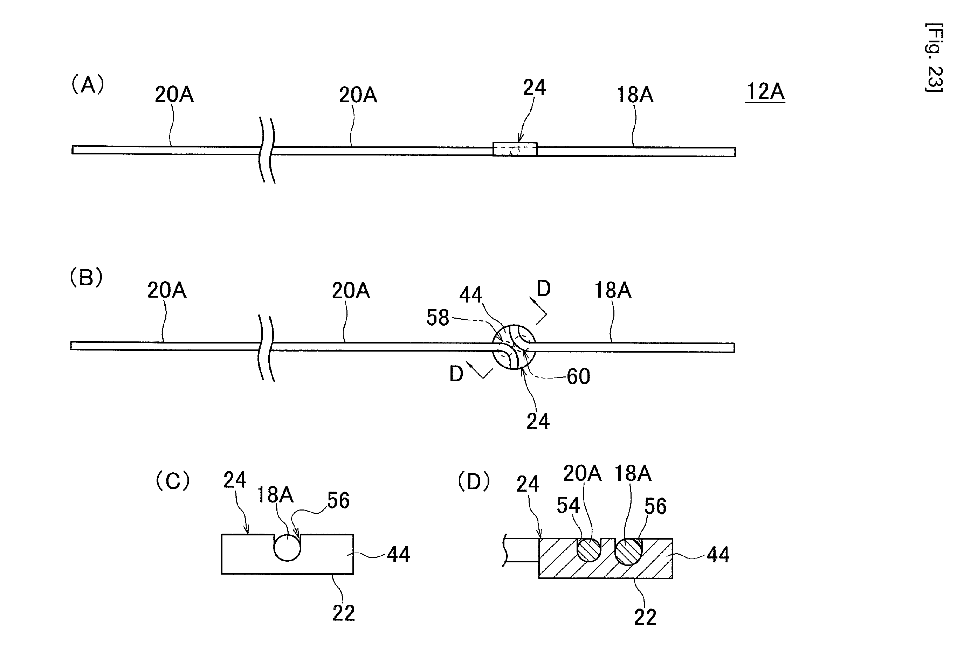

Figs. 23 are views illustrating still another example of the base material of the first correcting member illustrated in Fig. 1, in which Fig. 23A is a front view, Fig. 23B is a plan view, Fig. 23C is a right side view, and Fig. 23D is a sectional view on a line D-D in Fig. 23C.

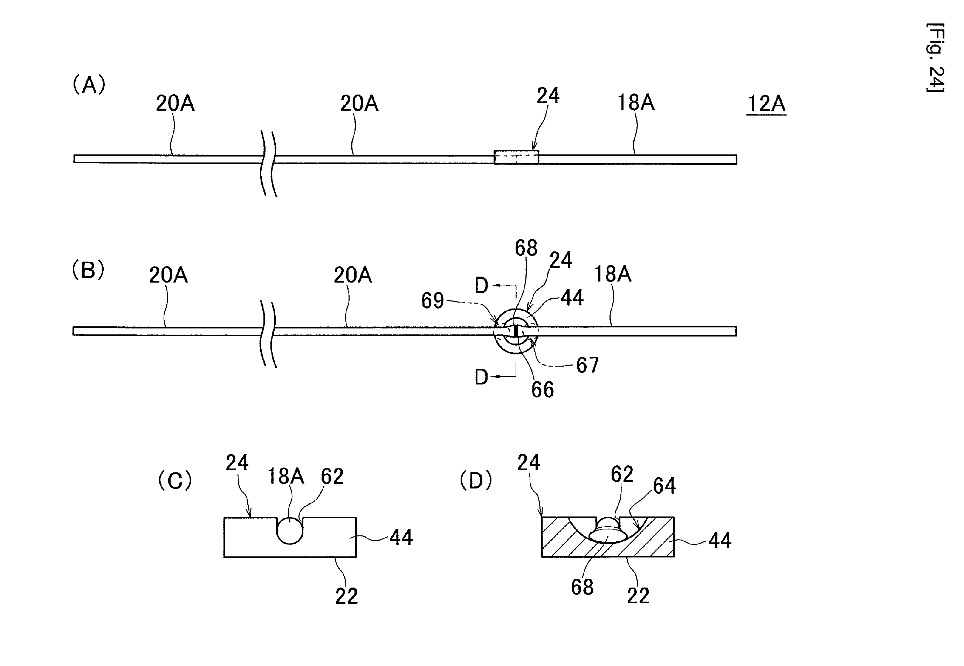

Figs. 24 are views illustrating still another example of the base material of the first correcting member illustrated in Fig. 1, in which Fig. 24A is a front view, Fig. 24B is a plan view, Fig. 24C is a right side view, and Fig. 24D is a sectional view on a line D-D in Fig. 24C.

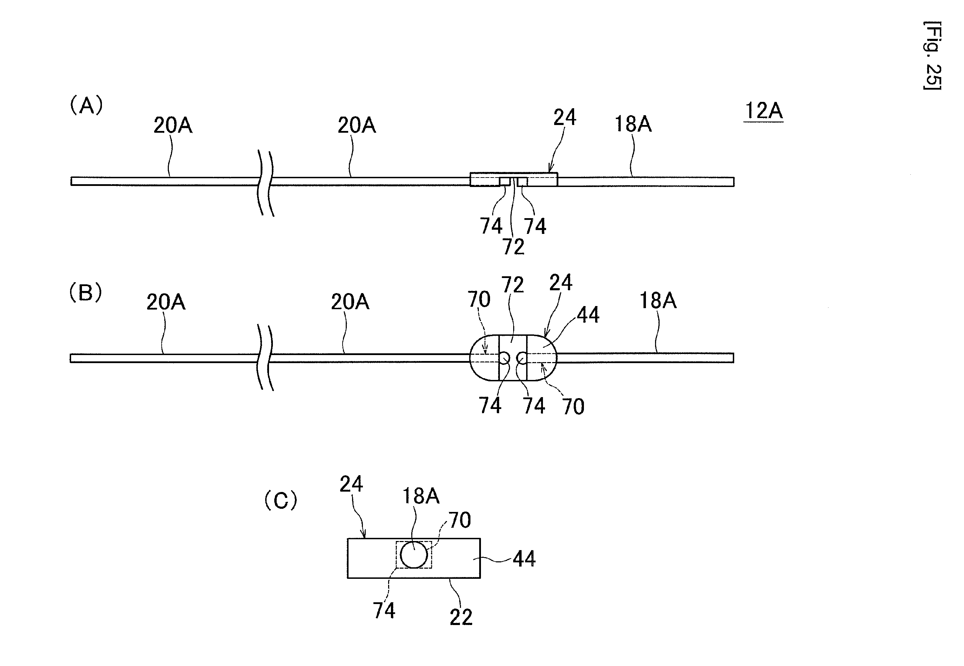

Figs. 25 are views illustrating another example of the base material of the first correcting member illustrated in Fig. 1, in which Fig. 25A is a front view, Fig. 25B is a plan view, and Fig. 25C is a right side view thereof.
Fig. 26 is a perspective view of an essential part illustrating an example of a prior-art nail corrective brace as a background of the present invention.
Fig. 27 is a perspective view of an essential part illustrating a process in the middle of mounting the prior-art corrective brace illustrated in Fig. 26 on the nail body.
Fig. 28 is a perspective view illustrating two components for forming a first correcting body, a second correcting body, and a correcting operation portion of the prior-art corrective brace illustrated in Fig. 26, that are a base material for the correcting body and a base material for the correcting operation portion.
Fig. 29 is a perspective view of an essential part illustrating a process of forming the first correcting body of the base material for the correcting body illustrated in Fig. 28.
Fig. 30 is another perspective view of an essential part illustrating a process of forming the first correcting body of the base material for the correcting body illustrated in Fig. 28.

### Reference Signs List

- 10: nail corrective brace
- 12: first correcting member
- 12A: base material for first correcting member
- 14: second correcting member
- 16: first locking piece
- 18: first correcting body
- 18A: hard line material
- 20: first correcting operation body
- 20A: soft line material
- 22, 23a, 23b: pressing surface
- 24: first support body
- 26: second locking piece
- 28: second correcting body
- 30: second correcting operation body
- 32: pressing surface
- 34: second support body
- 36: fixing portion
- 37: application portion
- 38: covering portion
- 40: first twisted portion
- 41: second twisted portion
- 42: first insertion ring
- 43: second insertion ring
- 42A: loop-shaped portion
- 44: first plate-shaped main body
- 46, 50, 54, 56, 62: first chamfered portion
- 48, 52, 58, 60, 67, 69: welded portion
- 49: decoration portion
- 49a: Swarovski
- 49b: light decoration piece
- 49c: artificial nail (gel nail)
- 64: first recess portion
- 66, 68: first diameter-enlarged flat portion
- 70: first tunnel hole
- 72: first stepped portion
- 74: first retaining portion
- 100: prior-art corrective brace
- 120: sandwiching means
- 140: twisting means
- 140a: hook
- 160, 180: sandwiching tool
- 200: cutting means
- 220: application tool
- A: nail body

### Description of Embodiments

A nail corrective brace 10 for a nail of an embodiment according to the present invention particularly includes a first correcting member 12 and a second correcting member 14 as illustrated in Figs. 17 and 18, for example.
The first correcting member 12 includes a first correcting body 18 formed of a hard line material having elasticity and provided with a first locking piece 16 locked by one side end edge in a width direction of a nail body A on the one end side thereof, a first correcting operation body 20 formed of a soft line material having elasticity and pulling the first correcting body 18 close to an intermediate portion side in the width direction of the nail body A with predetermined tension to pull up the one side end edge of the nail body A by the first locking piece 16, and a first support body 24 which connects the other end side of the first correcting body 18 and one end side of the first correcting operation body 20 and has a pressing surface 22 brought into contact with the surface of the nail body A to press the nail body A downward.

Similarly, the second correcting member 14 includes a second correcting body 28 formed of a hard line material having elasticity and provided with a second locking piece 26 locked by one side end edge in the width direction of the nail body A on the one end side tehreof, a second correcting operation body 30 formed of a soft line material having elasticity and pulling the second correcting body 28 close to the intermediate portion side in the width direction of the nail body A with predetermined tension to pull up the one side end edge of the nail body A by the second locking piece 26, and a second support body 34 which connects the other end side of the second correcting body 28 and one end side of the second correcting operation body 30 and has a pressing surface 32 brought into contact with the surface of the nail body A to press the nail body A downward.

Moreover, the corrective brace 10 includes a fixing portion 36 which adjusts the tension of pulling up the one end side and the other end side of the nail body A by crossing the other end side of the first correcting operation body 20 and the other end side of the second correcting operation body 30 with each other at the intermediate portion in the width direction of the nail body A and twisting and connects the first correcting operation body 20 and the second correcting operation body 30 in the vicinity of the surface of the nail body A while the predetermined tension is maintained.
Moreover, a covering portion 38 is formed so as to cover a fixing portion 36 and the periphery thereof on this fixing portion 36.

In the nail corrective brace 10 according to this embodiment, the first correcting member 12 and the second correcting member 14 have similar structures, actions and effects. Thus, particularly the first correcting member 12 will be described in detail in the detailed description of the embodiment illustrated below, while the second correcting member 14 will be described in brief or by omitting reference numerals.
Fig. 1 is a perspective view illustrating an example of the first correcting member 12 of the nail corrective brace 10 according to the present invention.
The first correcting member 12 includes the linear first correcting body 18 having a length direction thereof. The first correcting body 18 has the first locking piece 16 having a hook shape, for example, on one end side in the length direction. The first locking piece 16 and the first correcting body 18 are formed integrally of a stainless steel wire for spring or the like as a hard line material having elasticity.

Moreover, the first correcting member 12 includes the linear first correcting operation body 20 having a length direction. The first correcting operation body 20 is formed into a soft line having predetermined strength as a soft line material having elasticity by applying annealing processing and wire drawing processing to a stainless steel wire for spring or the like.
Furthermore, the first correcting member 12 includes the first support body 24 which supports the first correcting body 18 and the first correcting operation body 20. The first support body 24 is firmly connected to the other end side in the length direction of the first correcting body 18 and the one end side in the length direction of the first correcting operation body 20 and supports/connects the first correcting body 18 and the first correcting operation body 20.

Subsequently, an example of a connection structure of the first correcting body 18 and the first correcting operation body 20 at the first support body 24 will be described below in detail by referring to Fig. 2, for example.
Figs. 2 are views illustrating an example of the base material of the first correcting member illustrated in Fig. 1, in which Fig. 2A is a front view, Fig. 2B is a plan view, and Fig. 2C is a right side view thereof.
This base material 12A for the first correcting member includes a linear hard line material 18A for constituting the first correcting body 18 having the above-described first locking piece 16, a linear soft line material 20A for constituting the first correcting operation body 20, and the first support body 24. The line diameter of the hard line material 18A is formed having 0. 4 mm, for example, and the length of the hard line material 18A is formed having 12 mm, for example. The line diameter of the soft line material 20A is formed having 0.3 mm, for example, and the length of the soft line material 20A is formed having 52 mm, for example.
The first support body 24 includes a first plate-shaped main body 44 having a circular shape on plan view, for example, as illustrated in Figs. 2A, 2B, and 2C. A first chamfered portion 46 having a rectangular section, for example, is disposed from one end to the other end in the diameter direction of the first plate-shaped main body 44. The first plate-shaped main body 44 has one pressing surface 22 on one of its major surface sides and two pressing surfaces 23a and 23b on the other maj or surface side. The diameter of the first plate-shaped main body is formed having 2 mm, for example, a groove width of the first chamfered portion 46 is formed having approximately 0.7 mm, for example, and the groove depth is formed having approximately 0.4 mm, for example.

The other end side in the length direction of the above-described hard line material 18A and the one end side in the length direction of the soft line material 20A are fitted in the first chamfered portion 46 in a state adjacent to each other in parallel by the length of approximately 2 mm, for example. A welded portion 48 is formed by welding the other end side of the hard line material 18A, the one end side of the soft line material 20A and the first chamfered portion together at one to several spots. The welded portion 48 may be formed by separately welding the other end side of the hard line material 18A and the one end of the soft line material 20A to the first chamfered portion 46, respectively. As described above, the first correcting body 18 and the first correcting operation body 20 are firmly connected through the first support body 24.

On the other hand, a loop-shaped first insertion ring 42 is formed on the other end side in the length direction of the first correcting operation body 20 through a first twisted portion 40 as illustrated in Fig. 1. This first twisted portion 40 and the first insertion ring 42 are formed by inserting a hook 140a of twisting means 140, for example, through a loop-shaped portion 42A and rotating it while the loop-shaped portion 42A provided on the other end side in the length direction of the soft line material 20A illustrated in Fig. 2 is sandwiched by sandwiching means 120 such as nippers or the like as illustrated in Fig. 3. As described above, the first twisted portion 40 and the first insertion ring 42 are formed as illustrated in Fig. 4 on the base material 12A illustrated in Fig. 2.

Moreover, the first locking piece 16 is formed on the one end side in the length direction of the hard line material 18A of the base material 12A illustrated in Fig. 2. That is, the first support body 24 is sandwiched by a sandwiching tool 160 such as a needle holder or the like as illustrated in Fig. 5A and fixed. Then, a spot approximately 5 mm, for example, from the one end in the length direction of the hard line material 18A is sandwiched by a sandwiching tool 180 such as pliers or the like as illustrated in Figs. 5B and 5C and folded inward in the U-shape, so that a U-shaped portion 16A is formed. Further, a spot approximately 1 mm from the distal end of the U-shaped portion 16A is cut by cutting means 200 such as a cutter or the like as illustrated in Fig. 5D, so that the first locking piece 16 is formed as illustrated in Fig. 5E. As described above, the first correcting member 12 illustrated in Fig. 1 is formed.

In this embodiment, similarly to the above-described first correcting member 12, the second correcting member 14 constituting the second correcting body 28 having the second locking piece 26, the second correcting operation body 30, and the second support body 34 is formed. In this case, the other end side in the length direction of the second correcting body 28 and one end side in the length direction of the second correcting operation body 36 are firmly connected through the second support body 34. Moreover, a loop-shaped second insertion ring 43 is formed through a second twisted portion 41 on the second correcting operation body 30 as illustrated in Figs. 17 and 18, for example.

Subsequently, an example of a method (use method) of mounting the first correcting member 12 and the second correcting member 14 to the nail body A in order to correct deformation of a nail such as ingrown nails by using the first correcting member 12 and the second correcting member 14 of the nail corrective brace 10 according to this embodiment will be described below by referring to Figs. 6 to 16, 17, 18 and the like.
The first correcting member 12 and the second correcting member 14 are prepared. The nail body A is disinfected as appropriate for substantially the entirety thereof.
First, the first support body 24 of the first correcting member 12, for example, is sandwiched by a needle holder (not shown), for example, and the first locking piece 16 is locked at a spot approximately one third from the nail matrix (the root side of the nail) in a nail groove on one end edge side in the width direction of the nail body A as illustrated in Fig. 6. That is because, if the locked position of the first locking piece 16 is too close to the nail matrix (the root side of the nail), it might affect formation of the nail.
Similarly, on the other end edge side in the width direction of the nail body A, the second locking piece 26 of the second correcting member 14 is locked at a spot approximately one third from the nail matrix (the root side of the nail) in the nail groove on the other end edge side in the width direction of the nail body A as illustrated in Fig. 7.

Subsequently, the first locking piece 16 and the second locking piece 26 and their peripheries are initially and temporarily fixed by being held by fingertips of one hand, and the first correcting operation body 20 and the second correcting operation body 30 are crossed by the fingertips of the other hand and rotated once in a clockwise direction, for example, as illustrated in Fig. 8. At this time, the first support body 24 and the second support body 34 are brought into contact with the surface of the nail body A and supported, respectively.

Moreover, the hook 140a of the twisting means 140 hooks the first insertion ring 42 of the first correcting operation body 20 and the second insertion ring 43 of the second correcting operation body 30 as illustrated in Fig. 9 and subsequently, the hook 140a is wound up and twisted perpendicularly to the nail body A as illustrated in Fig. 10.

Then, after the first correcting operation body 20 and the second correcting operation body 30 are twisted as appropriate for a desired twisting amount (the number of twisting turns), the first correcting operation body 20 and the second correcting operation body 30 are inclined toward the toe tip side substantially at a right angle so as to conform to the nail body A as illustrated in Fig. 11.
Then, when a tension is applied to the first locking piece 16 and the second locking piece 26 through the first correcting operation body 20 and the second correcting operation body 30 and the locking pieces are tensed, the twisted portion (wound-up portion) is held by fingers and moved in the backwards and forwards direction of the nail body A as indicated by two-dot chain linear arrows in Fig. 12, to check firm tightening of the first correcting operation body 20 and the second correcting operation body 30.

If the first correcting member 12 and the second correcting member 14 are loose and tightened insufficiently, they are twisted and tightened again as indicated by rotating two-dot chain line arrows in Fig. 12. If the first correcting member 12 and the second correcting member 14 are integrally moved to the root side of the nail, it is determined that the first correcting member 12 and the second correcting member 14 have been correctly twisted.

Subsequently, when the first correcting member 12 and the second correcting member 14 have been correctly twisted and the twisting process is finished, the root side of the twisted portion (wound-up portion) is cut by cutting means 200 such as a cutter or the like as illustrated in Fig. 13. In this case, an extra portion of the twisted portion (wound-up portion) is cut off while two to three turns, for example, are left on the root side as illustrated in Fig. 14.
As a result, the fixing portion 36 which connects/fixes the first correcting operation body 20 and the second correcting operation body 30 in the vicinity of the surface of the nail body A is formed at the substantially center part in the width direction of the nail body A while a tension of pulling up the one end side and the other end side in the width direction of the nail body A is adjusted, and the predetermined tension is maintained as illustrated in Fig. 14.

At this time, the first correcting operation body 20 and the second correcting operation body 30 pull the first correcting body 18 and the second correcting body 28 close to the intermediate portion side in the width direction of the nail body A with predetermined tension, respectively, to pull up the one end edge portion and the other end edge portion of the nail body A by the first locking piece 16 and the second locking piece 26, respectively. In this case, the one end edge portion and the other end edge portion in the width direction of the nail body A are pulled in a direction (upward direction) opposite to the rolling direction of the ingrown nail and pulled up by the first correcting operation body 20 and the second correcting operation body 30.

Regarding the above-described tension, the longer the twisting length of the first correcting operation body 20 and the second correcting operation body 30 is, that is, the more they are twisted (the number of twisting turns), the larger this tension can be, while on the contrary, the shorter the twisting length is, that is, the less they are twisted (the number of twisting turns), the smaller the tension can be. This tension is adjusted as appropriate in accordance with the size and shape of the nail body A, the curved degree of the ingrown nail and the like.

In this embodiment, a paste-state ultraviolet curable resin material, for example, as a covering material is applied by an application tool 220 such as a brush or the like or is directly applied from a container (not shown) such as a tube containing the covering material or the like so as to cover the fixing portion 36 and the periphery thereof as illustrated in Fig. 15, and an application portion 37 is formed. In this case, if air bubbles enter the application portion 37, a crack or the like can easily occur in the application portion 37. Thus, the covering material is carefully applied so that the air bubbles do not enter when the covering material is applied by the application tool 220.
Furthermore, ultraviolet rays are projected to this application portion 37 on the entirety thereof by an ultraviolet LED (ultraviolet light emitting diode), for example, for predetermined time or 20 seconds, for example, as illustrated in Fig. 16. As a result, the application portion 37 is cured, and the covering portion 38 is formed as illustrated in Fig. 17.
This covering portion 38 protects the fixing portion 36 (connection end portion) which connects the first correcting operation body 20 and the second correcting operation body 30 in the vicinity of the surface of the nail body A and the periphery thereof and also makes the connecting force between the first correcting member 12 and the second correcting member 14 firmer. Moreover, it can stably maintain the connecting force.

Since this embodiment has a structure in which the first correcting operation body 20 and the second correcting operation body 30 are connected/fixed by being directly crossed with each other at the intermediate portion in the width direction of the nail body A and twisted as illustrated in Figs. 7 to 14 and the like, the tension can be increased as compared with the tool in which the tension acts through the ring-shaped correcting operation portion 128 as illustrated in Patent Document 1 (prior art). Therefore, the connecting force between the first correcting operation body 20 and the second correcting operation body 30 can be stably and continuously maintained. That is, the first correcting operation body 20 and the second correcting operation body 30 can be prevented from loosening and coming off from the nail body A as compared with that illustrated in Patent Document 1 (prior art).

Moreover, since this embodiment is not constructed such that the tension adjustment member 130 is hooked by the first connecting hook portion 126A and the second connecting hook portion 126B disposed in the upright state with respect to the surface of the nail body A as illustrated in Fig. 27 (prior art), for example, the height of the fixing portion 36 which becomes a connection end portion between the first correcting operation body 20 and the second correcting operation body 30 from the surface of the nail body A can be lowered as illustrated in Figs. 17 and 18. Therefore, it does not disturb daily life.

Moreover, in this embodiment, a pair of correcting members can be formed of two members, that is, the first correcting member 12 and the second correcting member 14 and the number of components is smaller than the tool requiring at least three members, that is, the first correcting body 120A, the second correcting body 120B, and the tension adjustment member 130 forming the ring-shaped correcting operation portion 128 as illustrated in Figs. 27 and 28 (prior art). Furthermore, in this embodiment, the number of machining processes including the process of curving, cutting, bending, folding and the like of the base line material for forming the first correcting member 120A and the second correcting member 120B is extremely fewer than the tool illustrated in Figs. 27 and 28 (prior art), and the machining processes are not complicated or require high-level technologies.
Thus, the method of mounting the first correcting member 12 and the second correcting member 14 on the nail body A in this embodiment is very simple, and the mounting time can be reduced extremely easily.

On the other hand, in the prior-art corrective brace 100, as illustrated in Fig. 28, arrangements and directions of the members constituting the first correcting body 120A and the second correcting body 120B are determined in advance, respectively, in the base material 102 for the correcting body, and if even only either one of the first correcting body 120A and the second correcting body 120B does not fit the nail body A, another base material 102 for the correcting body is required. On the contrary, in the corrective brace 10 according to this embodiment, each of the first correcting member 12 and the second correcting member 14 is formed of a single unit, and the directions and arrangements when being mounted on the nail body A are not determined in advance. In this case, the first correcting member 12 and the second correcting member 14 can replace each other in use. Thus, manufacturing costs of the first correcting member 12 and the second correcting member 14 can be lower than the prior-art corrective brace 100 and time required for mounting the first correcting member 12 and the second correcting member 14 on the nail body A can be further reduced.

Moreover, in this embodiment, in the first correcting member 12, the other end side of the first correcting body 18, the one end side of the first correcting operation body 20, and the first chamfered portion 46 are welded together at one to several spots, and the first correcting body 18 and the first correcting operation body 20 are firmly connected through the first support body 24. Since the same applies to the second correcting member 14, the second correcting body 28 and the second correcting operation body 30 are firmly connected through the second support body 34.
In this case, since a connection area between the first and second chamfered portions and the other end sides of the first and second correcting bodies as well as the one end sides of the first and second correcting operation bodies fitted in the first and second chamfered portions can be ensured wider than the connection by merely welding and connecting the other end sides of the first and second correcting bodies and the one end sides of the first and second correcting operation bodies, welding strength can be improved. Thus, the first and second correcting bodies and the first and second correcting operation bodies can be firmly connected to each other through the first and second support bodies.

Moreover, in this embodiment, the covering portion 38 is formed so as to cover the fixing portion 36 and thus, the amount of the covering material (an ultraviolet curable resin material, for example) used in the covering portion 38 can be smaller than the covering portion 134 formed over a wide range of the ring-shaped correcting operation portion 128, the first connecting hook portion 126A, and the second connecting hook portion 126B as illustrated in Fig. 26 (prior art), and a material cost can be lowered. Furthermore, since the height of the fixing portion 36 which becomes a connection end portion between the first correcting operation body 20 and the second correcting operation body 30 from the surface of the nail body A can be lowered, the use amount of the covering material used in the covering portion 38 can be further reduced by the height lowered, and the material cost of the covering portion 38 can be further lowered.
Also, in this embodiment, since the fixing portion 36 can be formed in an extremely compact form and smaller than that illustrated in Fig. 26 (prior art), for example, the first correcting operation body 20 and the second correcting operation body 30 can be mounted not only to the nail of the first toe but also to the nails of the second and other toes.

Moreover, in this embodiment, since the first support body 24 and the second support body 34 are brought into contact with the surface of the nail body A and supported, respectively, the first correcting body 18, the first correcting operation body 20, the second correcting body 28, and the second correcting operation body 30 are stably supported on the surface of the nail body A by the first support body 24 and the second support body 34, respectively, without shifting. In this case, as illustrated in Fig. 2, the pressing surface 22 on the one major surface (back face) side of the plate-shaped main body 44 is brought into contact with the surface of the nail body A and the one pressing surface 22 is formed having a plate shape. That is, since two pressing spots are formed on the first correcting member 12 and the second correcting member 14, the contact area with the nail body A becomes wider than the first correcting operation body 20 formed only of the hard line material 18A, the pressing force by the pressing surface 22 downward on the nail body A increases, and the first support body 24 and the second support body 34 can be supported on the surface of the nail body A further stably.
In this embodiment, the first correcting member 12 and the second correcting member 14 may be mounted on the nail body A so that the two pressing surfaces 23a and 23b on the other major surface (back face) side of the plate-shaped main body 44 are brought into contact with the surface of the nail body A, and the first support body 24 and the second support body 34 are supported on the surface of the nail body A. In this case, since four contact surfaces with the nail body A are formed with predetermined intervals by the first correcting member 12 and the second correcting member 14, the first support body 24 and the second support body 34 can be supported on the surface of the nail body A further stably.

Figs. 19 to 21 are perspective views of essential parts illustrating examples of modes in which various decoration technologies are applied to the covering portion 38 so as to form the decoration portions. In the mode example illustrated in Fig. 19, the decoration portion 49 may be formed by embedding or attaching a plurality of Swarovski 49a, for example, on the surface of the covering portion 38. The diameter of the Swarovski is preferably approximately 2 to 4 mm, for example. Moreover, as in the mode example illustrated in Fig. 20, a plurality of light decoration pieces 49b using a light reflection effect such as lames, spangles, hologram flakes and the like may be formed on the surface of the covering portion 38 or as in the mode example illustrated in Fig. 21, an artificial nail (gel nail) 49c may be further formed on the surface of the covering portion 38 by applying a gel-state artificial nail composition (gel-nail composition) containing an urethane resin and a photo-polymerizable monomer, for example, on the nail and then, by curing it through irradiation of ultraviolet rays.
In the embodiments illustrated in Figs. 19 to 21, various decoration technologies are applied to the covering portion 38 so as to form the decoration portion 49 and thus, the appearances are excellent, and particularly women can correct nails casually with a kind of a sense of enjoying fashion.

Figs. 22 to 25 are views illustrating other examples of the base material for the first correcting member. In the embodiment illustrated in Fig. 22, particularly the first chamfered portion 50 is formed having a U-shaped section as compared with the base material 12A illustrated in Fig. 2. A welded portion 52 is formed by the hard line material 18A and the soft line material 20A welded at one to several spots while the end portions in their axial directions (length directions) are abutted. The welded portion 52 may be formed by welding the vicinity of the abutted end portion of each of the hard line material 18A and the soft line material 20A to the first chamfered portion 46, separately. In this embodiment, the first and second correcting bodies 18 and 28 and the first and second correcting operation bodies 20 and 30 are firmly connected substantially linearly in their axial directions (length directions) through the first and second support bodies 24 and 34.

Moreover, in the embodiment illustrated in Fig. 23, particularly two first chamfered portions 54 and 56, each having a U-shaped section, for example, are formed in the first plate-shaped main body 44 as compared with the base material illustrated in Fig. 22, and the first chamfered portions 54 and 56 are formed, each having an R shape or L shape on plan view. In this case, the two first chamfered portions 54 and 56 are formed symmetrically in the radial direction so that the hard line material 18A and the soft line material 20A are arranged substantially linearly in their axial directions (length directions). Each of the other end side in the length direction of the hard line material 18A and the one end side in the length direction (axial direction) of the soft line material 20A is formed having an R shape or L shape on plan view, and welded portions 58 and 60 are formed by fitting them in the two first chamfered portions 54 and 56 and then, welding them together with the first chamfered portions 54 and 56 at one to several spots, respectively. In this embodiment, the first and second correcting bodies 18 and 28 and the first and second correcting operation bodies 20 and 30 are firmly connected substantially linearly in their axial directions (length direction) through the first and second support bodies 24 and 34 similarly to the embodiment in Fig. 22.

Moreover, in the embodiment illustrated in Fig. 24, the first support body 24 further includes a first recess portion 64 having an arc-shaped section, for example, communicating with a first chamfered portion 62 having a U-shaped section and formed in the first plate-shaped main body 44 as compared with the base material illustrated in Fig. 2. First diameter-enlarged flat portions 66 and 68 are disposed at the other end of the hard line material 18A and one end of the soft line material 20A, respectively. Welded portions 67 and 69 are formed by the first diameter-enlarged flat portions 66 and 68 fitted in the first chamfered portion 62 so that they are fitted in the first recess portion 64 and are welded at one to several spots together with the first chamfered portion 62 and the first recess portion 64, and.
Thus, in this embodiment, if the first diameter-enlarged flat portion is fitted in the first chamfered portion 62, the first diameter-enlarged flat portions 66 and 68 can be locked by the first recess portion 64. Thus, particularly, the other end of the first correcting body 18 and the one end of the first correcting operation body 20 can be easily fitted, positioned and welded in the first chamfered portion 62 as compared with that illustrated in Fig. 1, for example. In this embodiment, the first and second correcting bodies 18 and 28 and the first and second correcting operation bodies 20 and 30 are firmly connected to each other substantially linearly in their axial directions (length directions) through the first and second support bodies 24 and 34 similarly to the embodiment in Fig. 22.

Furthermore, in the embodiment illustrated in Fig. 25, the first support body 24 includes a first tunnel hole 70 penetrating from one end to the other end of the first plate-shaped main body 44 and a first stepped portion 72 crossing substantially at a right angle on plan view with the first tunnel hole 70. A first retaining portion 74 is disposed at the other end in the length direction of the hard line material 18A and at one end in the length direction of the soft line material 20, respectively. The one end side in the length direction of the hard line material 18A and the other end side of the soft line material 20 are inserted from the first stepped portion 72 side of the one first tunnel hole 70 and the other first tunnel hole 70 and come out of one end and the other end of the first plate-shaped main body 44, respectively, and welded to the first stepped portion 72 while the both first retaining portions 74 are locked by the peripheral edges of the first tunnel holes 70 on the first stepped portion 72 sides, respectively. In this case, the both first retaining portions 74 are welded to the first stepped portion 72, respectively, and welded portions (not shown) are formed.
Accordingly, in this embodiment, the other end of the first correcting body 18 and the one end of the first correcting operation body 20 can be more easily mounted on the first support body 24, positioned and welded as compared with the embodiment illustrated in Fig. 1, for example. After the welding, the first correcting body 18 and the first correcting operation body 20 can be retained and firmly connected to the first support body 24.

The first correcting member 12 according to each of the above-described embodiments includes the mode in which the first locking piece 16 is formed on the first correcting body 18 in advance as illustrated in Fig. 1, for example, and the mode in which the first locking piece 16 is formed on the first correcting body 18 afterwards as illustrated in Figs. 4 and 5, for example, and the same applies to the second correcting member 14. That is, the nail corrective brace 10 according to the present invention includes the first correcting member and the second correcting member having the both above-described modes.

## Claims

1. A nail corrective brace, comprising:
a first correcting member including:
a first correcting body formed of a hard line material having elasticity and provided with a first locking piece locked by one side end edge in a width direction of a nail body on one end side thereof;
a first correcting operation body formed of a soft line material having elasticity and pulling the first correcting body close to an intermediate portion side in the width direction of the nail body with predetermined tension to pull up the one side end edge of the nail body by the first locking piece; and
a first support body which connects the other end side of the first correcting body and one end side of the first correcting operation body and has a pressing surface brought into contact with a surface of the nail body to press the nail body downward;
a second correcting member including:
a second correcting body formed of a hard line material having elasticity and provided with a second locking piece locked by the other side end edge in the width direction of the nail body on the one end side thereof;
a second correcting operation body formed of a soft line material having elasticity and pulling the second correcting body close to the intermediate portion side in the width direction of the nail body with predetermined tension to pull up the other side end edge of the nail body by the second locking piece; and
a second support body which connects the other end side of the second correcting body and one end side of the second correcting operation body and has a pressing surface brought into contact with the surface of the nail body to press the nail body downward; and
a fixing portion which adjusts the tension of pulling up the one end side and the other end side in the width direction of the nail body by crossing the other end side of the first correcting operation body and the other end side of the second correcting operation body with each other at the intermediate portion in the width direction of the nail body and twisting and connects the first correcting operation body and the second correcting operation body in the vicinity of the surface of the nail body while the predetermined tension is maintained.

2. The nail corrective brace according to claim 1, wherein
the first support body includes a first plate-shaped main body and a first chamfered portion disposed in the first plate-shaped main body;
the other end side of the first correcting body and the one end side of the first correcting operation body are fitted in the first chamfered portion;
the first correcting body and the first correcting operation body are connected through the first support body by welding with the first chamfered portion or by welding the other end side of the first correcting body and the one end side of the first correcting operation body with the first chamfered portion together;
the second support body includes a second plate-shaped main body and a second chamfered portion disposed in the second plate-shaped main body;
the other end side of the second correcting body and the one end side of the second correcting operation body are fitted in the second chamfered portion; and
the second correcting body and the second correcting operation body are connected through the second support body by welding with the second chamfered portion or by welding the other end side of the second correcting body and the one end side of the second correcting operation body with the second chamfered portion together.

3. The nail corrective brace according to claim 2, wherein:
the first support body further includes a first recess portion communicating with the first chamfered portion;
a first diameter-enlarged flat portion is disposed at the other end of the first correcting body and one end of the first correcting operation body, respectively;
the other end side of the first correcting body and the one end side of the first correcting operation body are fitted in the first chamfered portion so that the first diameter-enlarged flat portions are both fitted in the first recess portion and are welded at least to the first chamfered portion and connected;
the second support body further includes a second recess portion communicating with the second chamfered portion;
a second diameter-enlarged flat portion is disposed at the other end of the second correcting body and one end of the second correcting operation body, respectively;
the other end side of the second correcting body and the one end side of the second correcting operation body are fitted in the second chamfered portion so that the second diameter-enlarged flat portions are both fitted in the second recess portion and are welded at least to the second chamfered portion and connected.

4. The nail corrective brace according to claim 1, wherein:
the first support body further includes a first tunnel hole penetrating the first support body from one end to the other end and a first stepped portion crossing the first tunnel hole substantially at a right angle on a plan view;
a first retaining portion is disposed at the other end of the first correcting body and the one end of the first correcting operation body, respectively;
the first correcting body and the first correcting operation body are inserted so that the sides opposite to the first retaining penetrate from the first stepped portion sides of one of the first tunnel holes and the other of the first tunnel holes to the one end and the other end of the first support body, respectively;
the first retaining portion is welded to the first stepped portion while being locked by a peripheral edge of the first tunnel hole on the first stepped portion side;
the second support body further includes a second tunnel hole penetrating the second support body from one end to the other end and a second stepped portion crossing the second tunnel hole substantially at a right angle on a plan view;
a second retaining portion is disposed at the other end of the second correcting body and the one end of the second correcting operation body, respectively;
the second correcting body and the second correcting operation body are inserted so that the sides opposite to the second retaining penetrate from the second stepped portion sides of one of the second tunnel holes and the other of the second tunnel holes to the one end and the other end of the second support body, respectively; and
the second retaining portion is welded to the second stepped portion while being locked by a peripheral edge of the second tunnel hole on the second stepped portion side.

5. The nail corrective brace according to any one of claims 1 to 4, further comprising:
a covering portion which covers the fixing portion.

6. The nail corrective brace according to claim 5, wherein
the covering portion includes a decoration portion to which a decoration technology is applied.
